# EUROPEAN PATENT SPECIFICATION

(11) **EP 1 242 096 B1**
(45) Date of publication and mention of the grant of the patent: **12.10.2005**
(21) Application number: 00981496.3
(22) Date of filing: 13.12.2000
(51) Int. Cl.: A61K 33/00, A61P 31/20

(54) **PHARMACEUTUCAL COMPOSITIONS FOR THE TREATMENT OF DNA VIRAL INFECTIONS COMPRISING A LOOP DIURETIC AND LITHIUM**
PHARMAZEUTISCHE ZUBEREITUNGEN ZUR BEHANDLUNG VON DNS VIRALER INFEKTIONEN, WELCHE EIN SCHLEIFENDIURETICUM UND LITHIUM ENTHALTEN
COMPOSITIONS PHARMACEUTIQUES POUR LE TRAITEMENT D'INFECTIONS VIRALES A ADN COMPRENANT UN DIURETIQUE DE L' ANSE ET DU LITHIUM

(30) Priority: 30.12.1999 GB 9930813; 12.01.2000 GB 0000683; 21.09.2000 GB 0023200
(43) Date of publication of application: 25.09.2002
(73) Proprietor: Henderson Morley Research and Development Limited, Birmingham B13 8JS (GB)
(72) Inventor: HARTLEY, Christopher, Edward, Halesowen B63 4QR (GB)
(74) Representative: Symons, Rupert Jonathan
(86) International application number: PCT/GB2000/004790
(87) International publication number: WO 2001/049300

(56) References cited:
- EP-A- 0 085 579
- EP-A- 0 135 312
- EP-A- 0 484 112
- WO-A-94/20119
- DE-A- 4 127 469
- SKINNER G.R.B. ET AL: "The effect of lithium chloride on the replication of Herpes simplex virus" MEDICAL MICROBIOLOGY AND IMMUNOLOGY, (1980) 168/2 (139-148). CODEN: MMIYAO, XP000901649
- SKINNER GR: "Lithium ointment for genital herpes [letter]" LANCET,XX,XX, vol. 2, no. 8344, 30 July 1983 (1983-07-30), page 288 XP002105266 ISSN: 0140-6736
- FELTEN G: "Erfahrungen mit Furosemid in der Behandlung des Zoster ophthalmicus." HAUTARZT, (1972 MAR) 23 (3) 140-2. , XP001058946

## Description

The invention relates to anti-viral compositions useful in the treatments and in particular to prophylactic and therapeutic treatments of DNA viral infections such as Herpes virus infections.

Herpes viruses are DNA viruses, having a central core of DNA within a proteinaceous structure. DNA carries the genetic code to reproduce the virus. Viruses must infect a living cell to reproduce. There are numerous viral proteins that are well characterised including important enzymes which act as ideal targets for antiviral chemotherapy. These include DNA polymerase and thymidine kinase which are needed for DNA replication. The replication of viral DNA is essential for virus infectivity. It is known that infecting viruses can alter the natural ionic balances of a living cell in the course of their replication.

The use of lithium in the treatment of DNA viral infections, and more specifically in the treatment of Herpes Simplex virus infections is disclosed in *Med Microbiol. Immunol. 108:139-148*. W0 00/10574 published after the date of the priority applications for this PCT application discloses the use of a loop diuretic in the treatment of a retrovirus, in this particular case to treat HIV infection. We have now unexpectedly discovered that the combined application of a loop diuretic and lithium gives an enhanced effect as compared to the administration of a loop diuretic or lithium alone.

By altering the cellular concentrations of ions, cellular ionic balances, cellular ionic milieu and cellular electrical potentials by the application of lithium and a loop diuretic it is possible to change the metabolism of the cell without detriment to the cell but so that virus replication is inhibited.

A first aspect of the invention provides the use of a loop diuretic and lithium for the manufacture of a medicament comprising a synergistic amount of loop diuretic and lithium for the treatment of DNA viral infections.

According to the invention in a second aspect there is provided a therapeutic composition useful in the treatment of DNA viral infections comprising a synergistic combination of a loop diuretic and lithium.

The synergistic application of a loop diuretic and lithium may be used in a method of treating a DNA viral infection.

The loop diuretic may be selected from a wide range of available agents. Preferably the loop diuretic is any one or more of frusemide, bumetanide, ethacrynic acid or torasemide. According to our studies the loop diuretics mediate their antiviral effects through alteration to the cellular concentration of ions, cellular ionic balances, cellular ionic milieu and electrical potentials.

Frusemide is an anthrilic acid derivative, chemically 4-chloro-N-furfuryl-5-sulfamoylanthranilic acid. It is practically insoluble in water at neutral pH, however is freely soluble in alkali. Frusemide exerts its physiological effect by inhibition of the transport of chloride ions across cell members. Frusemide is a loop diuretic with a short duration of action. It is used for treating oedema due to hepatic, renal, or cardiac failure and treating hypertension. The bioavailability of frusemide is between 60% to 70% and it is primarily excreted by filtration and secretion as unchanged drug. Frusemide acts on the Na+/K+/2Cl- cotransformer. For its diuretic effect, its predominant action is in the ascending limb of the loop of Henlé in the kidney. Loop diuretics markedly promote K⁺ excretion, leaving cells depleted in intracellular potassium. This may lead to the most significant complication of long term systemic frusemide usage namely a lowered serum potassium. We postulate that it is this action however which makes frusemide suitable for use as an agent against DNA viruses.

Recent evidence suggests that the major biotransformation product of frusemide is a glucuronide. Frusemide is extensively bound to plasma proteins, mainly albumin. Plasma concentrations ranging from 1 to 400 mcg/ml are 91-99% bound in healthy individuals. The unbound fraction ranges between 2.3-4.1% at therapeutic concentrations. The terminal half life of frusemide is approximately 2 hours, and it is predominantly excreted in the urine.

The lithium is preferably provided by a water-soluble or water miscible salt or ester, preferred are lithium chloride, lithium carbonate and lithium sulphate.

The loop diuretic and lithium may be applied simultaneously or slightly apart in time.

The invention is applicable to DNA viruses such as HSV1 and HSV2, CMV, VZV, and Pseudorabies. Other candidate viruses are parvoviruses; papoviruses; adenoviruses; hepadnoviruses and poxviruses.

The compositions of the invention may be adapted for external or internal administration. The formulations may be adapted for slow release. Topical and systemic applications are likely to be the most useful. Other ingredients may be present, provided that they do not compromise the anti-viral activity.

A preferred embodiment of this invention is the use of local concentrations of a loop diuretic and lithium as a highly effective treatment of virus infections of the eye. Recurrent Herpes infections of the cornea in man is the most common viral cause of blindness.

The use of contact lenses carrying e.g. impregnated with a loop diuretic and lithium would be a safe and efficient method for creating high intracellular concentrations to prevent or treat the disease. A depot application of a loop diuretic and lithium applied intra-occularly would be a suitable method for the treatment of cytomegalovirus retinitis, a major cause of blindness in patients suffering with AIDS.

Embodiments of the invention will now be described by way of illustration only with reference to the following examples:

### EXAMPLE 1

Compositions were applied to African green monkey kidney and BHK1 vero cells infected with type 2 herpes simplex virus strains 3345 and 180 at low, intermediate and high multiplicities of infection. MOI inhibition of virus replication was scored as follows:

| | |
|---|---|
| No inhibition | - |
| 20% inhibition | + |
| 40% inhibition | ++ |
| 60% inhibition | +++ |
| 80% inhibition | ++++ |
| 100% inhibition | +++++ |
| T denotes drug toxicity | |

Table 1 shows the results obtained for the application of frusemide and lithium chloride using African green monkey kidney cells and type 2 herpes simplex virus strain 3345.

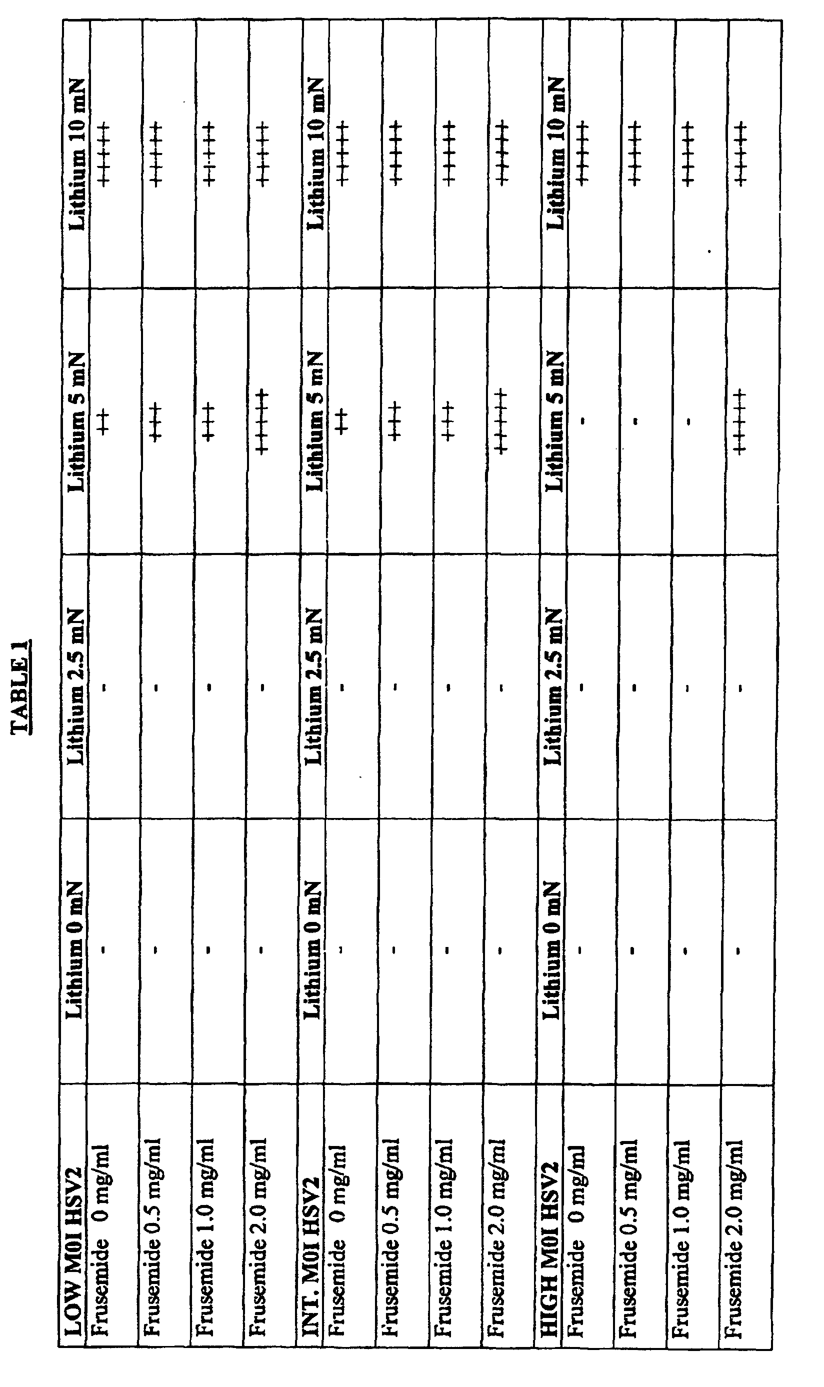

At a concentration of 10 mM the application of lithium chloride alone resulted in 100% inhibition of hsv2 replication at all multiplicities of infection. However, at this effective concentration lithium chloride is not suitable for continued application due to its toxicity.

There was no inhibition of hsv2 replication on application of frusemide at a concentration of 2 mg/ml at all multiplicities of infection.

100% inhibition of hsv2 replication was achieved using frusemide at an individually ineffective dose (2 mg/ml) in combination with lithium chloride at only 40% effective dose.

Similar results were obtained in experiments using other combinations of vero cells and type 2 herpes simplex strains.

### EXAMPLE 2

Bioassays with herpes simplex virus in vitro were undertaken to follow the anti-viral activity of the simultaneous administration of frusemide and lithium. Culture and assay methods follow those described by Lennette and Schmidt (1979) for herpes simplex virus and Vero cells with minor modifications.

### Herpes simplex strains used:

Type 1 herpes simplex strain HFEM is a derivative of the Rockerfeller strain HF (Wildy 1955), and Type 2 herpes simplex strain 3345, a penile isolate (Skinner et al 1977) were used as prototype strains. These prototypes were stored at -80°C until needed.

### Cell cultures:

African Green Monkey kidney cells (vero) were obtained from the National Institute of Biological Standards and Control UK and were used as the only cell line for all experiments in this example.

### Culture media:

Cells and viruses were maintained on Glasgows modified medium supplemented with 10% foetal bovine serum.

### Results:

### Inhibition of hsv2

| Multiplicity of infection (Dose of virus) | Effect of frusemide alone | Effect of lithium alone | Effect of frusemide and lithium in combination |
|---|---|---|---|
| High | - | - | ++++ |
| Medium | ++ | - | ++++ |
| Low | ++ | ++ | ++++ |

Our evaluations indicate that an enhanced anti-viral effect takes place with the simultaneous administration of a diuretic e.g. frusemide (1mg/ml) and lithium (10mM). Virus activity was prevented by applying a low concentration of the stock solution to vero cells infected with hsv2, and the anti-viral effect of the stock solution was far greater than the effects of lithium or frusemide applied alone.

The same experiments produced comparable results when applied to hsv1 infected Vero cells (strains HFEM and KOS).

These examples demonstrate the advantageous effects of combining a loop diuretic and lithium salt at individually ineffective doses.

## Claims

1. Use of a loop diuretic and lithium for the manufacture of a medicament comprising a synergistic amount of loop diuretic and lithium for the treatment of DNA viral infections.

2. A therapeutic composition useful for the treatment of DNA viral infections comprising a synergistic combination of a loop diuretic and lithium.

3. A composition according to Claim 2 adapted for topical application.

4. A composition according to Claim 2 adapted for systemic application.

5. A composition according to Claim 3, comprising 1mg/ml of a loop diuretic and 10mM solution of lithium salt.

6. A composition according to Claim 3, comprising 2mg/ml of a loop diuretic and 5mM solution of lithium salt.

7. A composition according to Claim 3, adapted for intra-occular depot application.

8. A composition according to any preceding Claim, wherein the loop diuretic is frusemide.

9. Contact lenses carrying, e.g. impregnated with, a loop diuretic and lithium arranged to deliver, in use, a synergistic combination of the loop diuretic and lithium to treat a DNA viral infection of the eye.

## Patentansprüche

1. Verwendung von einem Schleifendiuretikum und Lithium zur Herstellung eines Medikaments, umfassend eine synergistische Menge an Schleifendiuretikum und Lithium, zur Behandlung von DNA-Virus-Infektionen.

2. Therapeutische Zusammensetzung, geeignet zur Behandlung von DNA-Virus-Infektionen, umfassend eine synergistische Kombination von einem Schleifendiuretikum und Lithium.

3. Zusammensetzung nach Anspruch 2, adaptiert für die topische Anwendung.

4. Zusammensetzung nach Anspruch 2, adaptiert für die systemische Anwendung.

5. Zusammensetzung nach Anspruch 3, umfassend 1 mg/ml eines Schleifendiuretikums und eine 10 mM Lösung eines Lithiumsalzes.

6. Zusammensetzung nach Anspruch 3, umfassend 2 mg/ml eines Schleifendiuretikums und eine 5 mM Lösung eines Lithiumsalzes.

7. Zusammensetzung nach Anspruch 3, adaptiert für die intraokulare Depotanwendung.

8. Zusammensetzung nach irgendeinem der vorhergehenden Ansprüche, wobei das Schleifendiuretikum Frusemid ist.

9. Kontaktlinsen, die ein Schleifendiuretikum und Lithium in einer Anordnung zur Abgabe, beim Gebrauch, einer synergistischen Kombination von dem Schleifendiuretikum und Lithium zur Behandlung einer DNA -Virus-Infektion des Auges tragen, z.B. damit imprägniert sind.

## Revendications

1. Utilisation d'un diurétique de l'anse et de lithium pour la fabrication d'un médicament comprenant une quantité synergique de diurétique de l'anse et de lithium pour le traitement d'infections virales de l'ADN.

2. Composition thérapeutique utile pour le traitement d'infections virales de l'ADN comprenant une combinaison synergique d'un diurétique de l'anse et de lithium.

3. Composition selon la revendication 2, adaptée pour une application topique.

4. Composition selon la revendication 2, adaptée pour une application systémique.

5. Composition selon la revendication 3, comprenant 1 mg/mL d'un diurétique de l'anse et une solution de sel de lithium 10 mM.

6. Composition selon la revendication 3, comprenant 2 mg/mL d'un diurétique de l'anse et une solution de sel de lithium 5 mM.

7. Composition selon la revendication 3, adaptée pour une application retard intraoculaire.

8. Composition selon l'une quelconque des revendications précédentes, dans laquelle le diurétique de l'anse est le frusémide.

9. Lentilles de contact portant, par exemple imprégnées d'un diurétique de l'anse et de lithium arrangées pour délivrer, durant l'utilisation, une combinaison synergique du diurétique de l'anse et du lithium pour traiter les infections virales de l'ADN des yeux.
